# EUROPEAN PATENT SPECIFICATION

(11) **EP 3 643 327 B1**
(45) Date of publication and mention of the grant of the patent: **24.01.2024**
(21) Application number: 19205197.7
(22) Date of filing: 24.10.2019
(51) Int. Cl.: A61L 2/20, C01B 15/027, G01N 21/00

(54) **APPARATUS AND METHOD TO INTRODUCE VAPORISED HYDROGEN PEROXIDE AT LOW CONCENTRATION INTO AN ISOLATOR**
VORRICHTUNG UND VERFAHREN ZUR EINFÜHRUNG VON VERDAMPFTEM WASSERSTOFFPEROXID IN NIEDRIGER KONZENTRATION IN EINEN ISOLATOR
APPAREIL ET MÉTHODE POUR INTRODUIRE DU PEROXYDE D'HYDROGÈNE VAPORISÉ À FAIBLE CONCENTRATION DANS UN ISOLATEUR

(30) Priority: 24.10.2018 IT 201800009752
(43) Date of publication of application: 29.04.2020
(73) Proprietor: Comecer S.p.A., 48014 Castel Bolognese (IT)
(72) Inventor: GUIDI, Giacomo, 48014 CASTEL BOLOGNESE (RA) (IT); ZANELLI, Alessia, 48014 CASTEL BOLOGNESE (RA) (IT)
(74) Representative: Studio Torta S.p.A.

(56) References cited:
- WO-A1-2018/160412
- US-A- 5 847 393
- US-A1- 2009 074 611
- THORPE M J ET AL: "Broadband cavity ringdown spectroscopy for sensitive and rapid molecular detection", SCIENCE, AAAS, AMERICAN ASSOC. FOR THE ADVANCEMENT OF SCIENCE, US, vol. 311, no. 5767, 17 March 2006 (2006-03-17), pages 1595-1599, XP002433363, ISSN: 0036-8075, DOI: 10.1126/SCIENCE.1123921

## Description

### TECHNICAL FIELD

The present invention concerns an apparatus and a corresponding method for introducing low-concentration vaporized hydrogen peroxide into an isolated chamber.

In particular, the present invention is advantageously, but not exclusively, applied in a pharmaceutical isolator, to which the following description will make explicit reference without loss of generality.

### BACKGROUND ART

Apparatuses are known for introducing hydrogen peroxide in the form of vapour or atomized into a manipulation chamber of a pharmaceutical isolator in order to carry out a decontamination cycle that reduces the microbiological load in the chamber. In the above-mentioned apparatuses, the hydrogen peroxide to be introduced into the chamber is typically obtained by vaporization or atomization of an aqueous solution of hydrogen peroxide in a percentage ranging from 8% to 50%.

The technique of generation by means of vaporization entails dripping the aqueous solution of hydrogen peroxide onto a hot plate, so as to instantly vaporize the hydrogen peroxide and transfer the vaporized hydrogen peroxide (VHP), also called hydrogen peroxide vapour (HPV), from the vaporization point to the chamber to be decontaminated through a forced ventilation system, for example one which generates a laminar air flow in the chamber. This technique is also called "flash vaporization", since it produces a phase (state) change in the hydrogen peroxide from liquid to vapour at a speed of several millilitres per minute. The technique of vaporization is particularly suited to the decontamination of isolators included in pharmaceutical production lines that comply with the GMP regulations.

The technique of generation by means of atomization entails atomizing the hydrogen peroxide directly in the chamber. The hydrogen peroxide does not change phase, but remains in very fine liquid particles, namely micro-drops with dimensions indicatively ranging between 0.5 and 10 pm, and is often called dry fog. The atomization technique does not require a ventilation system and allows very rapid decontamination due to the capacity of the micro-drops to easily capture the particles of contaminating agents.

A decontamination cycle of an isolated chamber comprises essentially three steps:
- conditioning, wherein the vaporized or atomized hydrogen peroxide is introduced into or generated at high concentration in the chamber to be decontaminated;
- reduction, wherein the hydrogen peroxide is maintained stable at high concentrations for a time necessary to guarantee reduction of the microbiological load in the chamber; and
- aeration, wherein the hydrogen peroxide is removed from the chamber so as to restore the initial and normal working conditions in the chamber.

During the conditioning step the concentration of the vaporized or atomized hydrogen peroxide can reach 1500 PPM (parts per million), whereas at the end of the aeration step the concentration should drop to below 1 PPM, which is the acceptable limit in an environment in which an operator is present.

In today's pharmaceutical production world, the sector of biological drugs is expanding, namely drugs whose production process requires the presence and/or the manipulation of biological material, which is sensitive to oxidizing molecules such as hydrogen peroxide. For example, in the production of vaccines and advanced therapeutic medicinal products (ATMPs) biological material is manipulated, such as cells, bacteria or viruses, which is highly sensitive to hydrogen peroxide and begins to deteriorate at hydrogen peroxide concentrations of a few dozen PPB (parts per billion). The concentration of hydrogen peroxide at the end of a decontamination cycle (1 PPM) is approximately one hundred times greater than the concentration required to avoid damage to biological drugs.

A decontamination cycle that ends with a hydrogen peroxide concentration of 1 PPM normally has a duration of approximately 1 hour. To obtain a final concentration much lower than 1 PMM in order to be sure of not damaging the biological drugs during their manipulation or production in the isolator chamber would require a much longer and more complex aeration step which would result in the decontamination cycle having an overall duration of up to 24 hours. This duration would cause a drastic reduction in the throughput of the production line which comprises the pharmaceutical isolator to be decontaminated.

It is therefore important to know with a certain accuracy the degree of damage of a biological drug according to the variation in the concentration of the residual hydrogen peroxide at the end of a decontamination cycle, to identify the best compromise between residual concentration and duration of the aeration step in order to guarantee an acceptable mean throughput for the production line. However, the apparatuses known for the generation of vaporized or atomized hydrogen peroxide do not allow the performance of tests to verify the effects of the hydrogen peroxide residue on the biological material, since said apparatuses are not able to generate and maintain, in a reduced volume such as that of an isolator chamber, vaporized hydrogen peroxide which has a concentration value ranging from 1 PPM to 10 PPM and is sufficiently stable in the long term.

United States Patent No. 5,847,393 discloses an essentially continuous measurements of hydrogen peroxide vapour in the presence of water vapour by means of near infrared (NIR) spectroscopy using fibre optic cables to transmit infrared radiation between a sterilization chamber and the NIR instrument. The measurement of hydrogen peroxide concentration is incorporated into a control system which automatically adds gaseous hydrogen peroxide to the sterilization chamber when the measured concentration falls below a pre-calculated value. Such a control system ensures the presence in the sterilization chamber of an adequate concentration of gaseous hydrogen peroxide to effect sterilization throughout the sterilization procedure.

United States Patent Application No. US2009/0074611A1 discloses a photolytic hydrogen peroxide generator (10), which converts water to activated oxygen for electrolyte absorption, regulates pH, removes hydrogen and other gases and includes a photolytic cell (16) where chemical reactions occur.

Article by Michael J. Thorpe et al., titled "Broadband Cavity Ringdown Spectroscopy for Sensitive and Rapid Molecular Detection" and published on March 2006, discloses an efficient cavity ringdown spectroscopy in which a broad-bandwidth optical frequency comb is coherently coupled to a high-finesse optical cavity that acts as the sample chamber. Real-time quantitative measurements were made for molecules such as C₂H₂, O₂, H₂O, and NH₃. WO 2018/160412 A1 discloses an apparatus to deliver vaporised hydrogen peroxide in a concentration up to 0.45 ppm to a closed environment.

### DISCLOSURE OF INVENTION

The object of the present invention is to provide a method for introducing, into an isolated chamber, low-concentration vaporized hydrogen peroxide which is stable in the long term, said apparatus being without the drawbacks described above and, at the same time, easy and inexpensive to produce.

In accordance with the present invention an apparatus according to claim 1 and a method according to claim 8 are provided for introducing vaporized hydrogen peroxide at a concentration lower than or equal to 10 PPM into an isolated chamber, and in particular into a chamber of an isolator, and an isolator according to claim 4.

### BRIEF DESCRIPTION OF THE DRAWINGS

The present invention will now be described with reference to the attached drawings, which illustrate a non-limiting embodiment example thereof, in which:
- figure 1 illustrates, by means of a simplified block diagram, a pharmaceutical isolator comprising an apparatus for introducing low-concentration vaporized hydrogen peroxide into a chamber of the isolator, the apparatus of which is produced according to the present invention; and
- figure 2 illustrates a block diagram of a further embodiment of the isolator of figure 1.

### BEST MODE FOR CARRYING OUT THE INVENTION

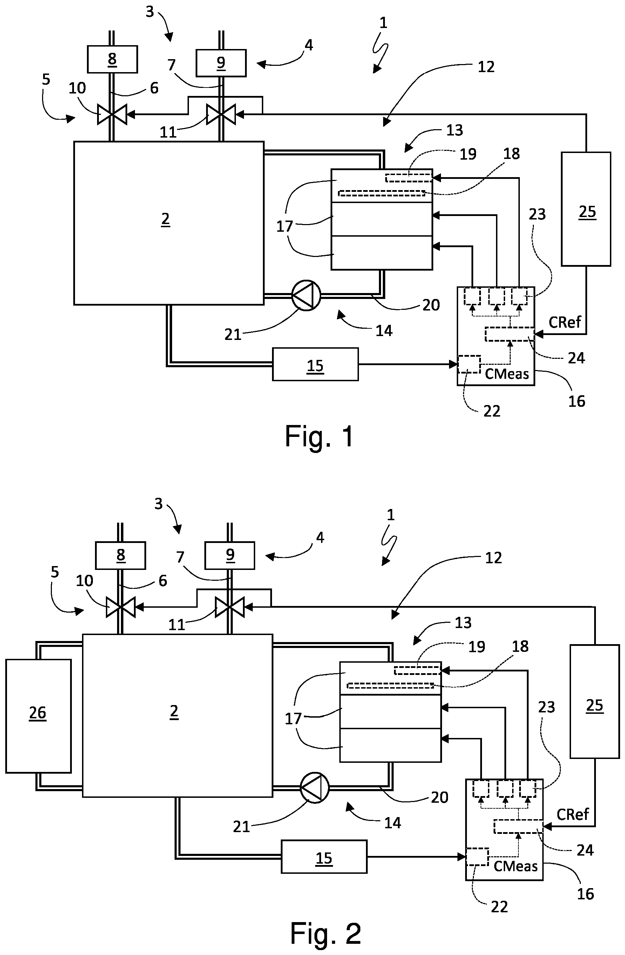

In figure 1, the number 1 generically indicates, overall, a pharmaceutical isolator comprising an isolated chamber 2, inside which it is possible to manipulate and/or dispense drugs in aseptic or non-aseptic conditions through glove flanges, known per se and not illustrated, hermetically fixed to a door with hermetic seal, also not illustrated, of the isolated chamber 2. The isolated chamber 2 has a volume indicatively ranging from 0.3 to 5 m³. The isolated chamber 2 is defined by a casing having rigid walls, which are made of materials adapted to pharmaceutical processes and hygienically designed. The isolator 1 comprises a forced ventilation system 3 to circulate air in the isolated chamber 2, preferably in the form of laminar flow, for the purpose, as is known, of guaranteeing sterile conditions. The forced ventilation system 3 comprises an air filtering system 4 of the type comprising one or more HEPA (High Efficiency Particulate Absorber) filters, otherwise known as "absolute filters", to drastically limit the presence of particulate matter in the isolated chamber 2, and a system of valves 5 to allow or block the circulation of air in the isolated chamber 2.

In particular, the forced ventilation system 3 comprises a delivery duct 6 and a return duct 7 for respectively introducing air into and extracting air from the isolated chamber 2. The air filtering system 4 comprises at least two HEPA filters 8 and 9, preferably in class H14, the first one being arranged in the delivery duct 6 and the second one in the return duct 7. The system of valves 5 comprises two valves 10 and 11, a first one being arranged in the delivery duct 6 and the second one in the return duct 7. As is known, class H14 corresponds to a filtering efficiency higher than 99.995%.

The isolator 1 comprises an apparatus 12 for introducing low-concentration vaporized hydrogen peroxide, namely a concentration lower than or equal to 10 PPM, into the isolated chamber 2. The apparatus 12 comprises: a generator of vaporized hydrogen peroxide, shortened to VHP generator below and indicated by 13 in figure 1, operating by means of photocatalysis; a closed-path forced ventilation system 14 for introducing into the isolated chamber 2 the vaporized hydrogen peroxide produced by the VHP generator 13; a laser spectroscopy gas analyser 15, which communicates with the isolated chamber 2 for measuring the concentration of the vaporized hydrogen peroxide in the isolated chamber 2; and a control unit 16 configured for controlling the VHP generator 13 as a function of measured values of the vaporized hydrogen peroxide concentration, indicated by CMeas in figure 1, and at least one reference value of the vaporized hydrogen peroxide concentration, indicated by CRef in figure 1.

The VHP generator 13 comprises one or more generator modules 17, each of which comprises a respective metal plate 18 having a coating of photocatalytic material activatable by UV light to transform the humidity of the air into vaporized hydrogen peroxide and a respective UV radiation source 19 arranged so as to irradiate UV radiation on the coating of the plate 18 thus activating the photocatalysis. Preferably, the photocatalytic material consists of titanium dioxide. Preferably, the UV radiation source 19 is a UV lamp.

The forced ventilation system 14 comprises a closed pneumatic circuit 20 for establishing communication between the isolated chamber 2 and the VHP generator 13 and a fan 21 positioned in the pneumatic circuit 20 to circulate the air between the isolated chamber 2 and the VHP generator 13. In this way, the humidity of the air present in the isolated chamber 2 reaches the VHP generator 13 where it is transformed into vaporized hydrogen peroxide through photocatalysis.

The gas analyser 15 carries out a cavity ring-down spectroscopy (CRES). For example, the gas analyser 15 is the model PI2114 produced by Picarro Inc..

The control unit 16 is configured to control the power of the UV radiation sources 19 so as to adjust the intensity of the UV radiation and therefore the quantity of vaporized hydrogen peroxide generated and introduced into the isolated chamber 2.

In particular, the control unit 16 comprises an appropriately configured PLC device. In greater detail, the PLC device implements a signal conditioning module 22 which processes a signal supplied by the gas analyser 15 to obtain measured concentration values, one or more driver modules 23, each of which is associated with a respective generator module 17 to power the respective UV radiation source 19, and a signal processing module 24 to compare the measured concentration values with a reference concentration value.

The isolator 1 comprises an electronic control unit 25 to control various controllable devices of the isolator 1, for example the valves 10 and 11, and to program operation of the isolator 1, for example to set the concentration reference value CRef of the vaporized hydrogen peroxide used by the control unit 16.

The VHP generator 13 based on photocatalysis and the feedback control of the same based on concentration measurements carried out by means of the laser spectroscopy gas analyser 15 allow the vaporized hydrogen peroxide to be maintained at very low concentration values in the isolated chamber 2, in particular comprised between 1 PPB and 10 PPM.

In fact, the apparatus 12 implements a method for introducing low-concentration vaporized hydrogen peroxide into the isolated chamber 2, comprising the steps of generating the vaporized hydrogen peroxide by means of photocatalysis, introducing the vaporized hydrogen peroxide into the isolated chamber 2 by means of forced ventilation, measuring the concentration of vaporized hydrogen peroxide in the isolated chamber 2 by means of the spectroscopy gas analyser 15, and adjusting the generation of the vaporized hydrogen peroxide as a function of the concentration measured values CMeas and at least one concentration reference value CRef.

Advantageously, generation and introduction of the hydrogen peroxide into the isolated chamber 2 is preceded by a step of forced circulation of air in the isolated chamber 2 for a predetermined time, carried out by means of the forced ventilation system 3 with the valves 10 and 11 open. The air filtering system 4 will limit the particulate matter in the air present in the isolated chamber 2. At the end of the predetermined time, the valves 10 and 11 are closed and subsequently the apparatus 12 for introducing the vaporized hydrogen peroxide into the isolated chamber 2 is activated.

The measurement of the concentration of vaporized hydrogen peroxide is all the more accurate the cleaner the air in the isolated chamber 2, and in particular when the particulate matter is reduced to very low quantities, especially when it is necessary to maintain a very low concentration value, namely comprised between 1 PPB and 10 PPM. Assuming we set a concentration reference value CRef which is constant over time, greater accuracy of the concentration measurement corresponds to greater stability of the vaporized hydrogen peroxide concentration in the isolated chamber 2.

The isolator 1 described above allows tests to be carried out to verify the effects of the hydrogen peroxide residue on a biological material, for example a long duration test, maintaining the concentration reference value CRef constant, to evaluate the final effects of any hydrogen peroxide residue on the biological material, or a dynamic test, varying the concentration reference value CRef for example according to a decreasing slope, to simulate the final step of a decontamination cycle.

According to a further embodiment illustrated in figure 2, in which the corresponding elements are indicated by the same numbers and letters as figure 1, the isolator 1 comprises an apparatus 26 for introducing into the isolated chamber 2 high-concentration vaporized hydrogen peroxide, namely a concentration greater than or equal to 400 PPM, preferably ranging from 400 PPM to 1500 PPM, to allow microbiological decontamination of the isolated chamber 2. The apparatus 26 comprises a vaporized or atomized hydrogen peroxide generator of a known type, for example of the flash vaporization or dry fog type. The apparatus 26 is therefore adapted to be used in a decontamination cycle.

The isolator 1 in the embodiment of figure 2 can be used in the more conventional manner, namely to manipulate drugs with the possibility of periodically carrying out decontamination cycles, due to the presence of the apparatus 26, but also in an alternative manner, namely to carry out tests to verify the effects of hydrogen peroxide residue on a biological material, due to the presence of the apparatus 12. It should be noted that the aseptic conditions in the chamber 2 are achieved due to the circulation of air according to a laminar flow and to the simultaneous introduction of a high concentration of vaporized hydrogen peroxide.

It should be further noted that the apparatus 12 is also suitable for an isolator not strictly for pharmaceutical use, for example an isolator for the food industry or for regenerative medicine, or for an aseptic area with restricted access, also known as Restricted Access Barrier System (RABS), namely in all plants that comprise a chamber or an isolated environment in which it is necessary to reduce the microbiological load in order to then restore the initial and normal working conditions in the chamber.

In short, the apparatus 12 is suitable for utilization in an isolator for generic use comprising: an isolated chamber, which is defined by a casing having rigid walls and is provided with a hermetically sealed door; a forced ventilation system to create a one-way flow of air substantially in the entire volume of the isolated chamber; and an air filtering system, preferably of HEPA type, coupled with or integrated in the forced ventilation system to limit the presence of particulate matter in the isolated chamber. A pharmaceutical type isolator has the following further characteristics: the walls of the isolated chamber are made of material suitable for pharmaceutical use, namely antibacterial or easily washable, the air flow in the isolated chamber is laminar and is kept at a pressure greater than that of the air outside the isolated chamber to avoid the entry of decontaminated air, the door comprises glove flanges for manipulating drugs inside the isolated chamber.

Although the invention described above refers in particular to a precise embodiment example, it should not be considered limited to said embodiment example, since all the variations, modifications or simplifications covered by the attached claims fall within its scope.

## Claims

1. An apparatus for introducing vaporized hydrogen peroxide at a concentration smaller than or equal to 10 PPM into an isolated chamber, and in particular into a chamber of an isolator, for example a pharmaceutical isolator, the apparatus comprising: a vaporized hydrogen peroxide generator (13), which operates through photocatalysis and comprises at least one plate (18) having a coating of photocatalytic material activatable by UV radiation, in order to transform the humidity of the air present in the isolated chamber (2) into vaporized hydrogen peroxide, and at least one UV radiation source (19) arranged so as to irradiate UV radiation on said coating, thus activating the photocatalysis; and a forced ventilation system (14), which is suitable to introduce the vaporized hydrogen peroxide into the isolated chamber (2)and comprises a pneumatic circuit (20) suitable to establish a closed-path communication between the isolated chamber (2) and the vaporized hydrogen peroxide generator (13) and a fan (21) positioned in the pneumatic circuit (20) to circulate the air between the isolated chamber (2) and the vaporized hydrogen peroxide generator (13); the apparatus being **characterized in that** said plate is a metal plate (1) and **in that** it comprises a spectroscopy gas analyser (15), which is suitable to communicate with the isolated chamber (2) so as to measure the concentration of the vaporized hydrogen peroxide in the isolated chamber (2), and control means (16), which are configured to control the vaporized hydrogen peroxide generator (13) as a function of measured values (CMeas) of the vaporized hydrogen peroxide concentration and of at least one reference value (CRef) of the vaporized hydrogen peroxide concentration and to control the power of said UV radiation source (19) so as to adjust the intensity of the UV radiation and, hence, the quantity of vaporized hydrogen peroxide generated and introduced into the isolated chamber (2).

2. The apparatus according to claim 1, wherein said photocatalytic material consists of titanium dioxide.

3. The apparatus according to claim 1 or 2, wherein said spectroscopy gas analyser (15) carries out a CRDS spectroscopy.

4. An isolator, in particular a pharmaceutical isolator, comprising an isolated chamber (2) and an apparatus (12) for introducing vaporized hydrogen peroxide at a concentration lower than or equal to 10 PPM into the isolated chamber (2) according to any one of the claims from 1 to 3.

5. The isolator according to claim 4 and comprising a further forced ventilation system (3), which is designed to allow air to circulate in the isolated chamber (2) and comprises HEPA air filtering means (4) to limit the presence of particulate matter in the isolated chamber (2), valves (10, 11) to allow or prevent said air from circulating in the isolated chamber (2), and an electronic control unit (25) configured to close the valves (10, 11) before activating said apparatus (12).

6. The isolator according to claim 5, wherein said further forced ventilation system (3) comprises a delivery duct (6) and a return duct (7) to introduce air into and extract air from said isolated chamber (2), respectively, said HEPA air filtering means (4) comprising at least two HEPA filters (8, 9), preferably belonging to class H14, which are arranged, a first one, in the delivery duct (6) and, the second one, in the return duct (7), and said valves comprise two valves (10, 11), which are arranged, a first one, in the delivery duct (6) and, the second one, in the return duct (7).

7. The isolator according to any one of the claims from 4 to 6 and comprising a further apparatus (26) to introduce into the isolated chamber (2) vaporized hydrogen peroxide at a concentration higher than or equal to 400 PPM, to allow for a microbiological decontamination of the isolated chamber (2).

8. A method to introduce vaporized hydrogen peroxide at a concentration smaller than or equal to 10 PPM into an isolated chamber, and in particular into a chamber of an isolator, for example a pharmaceutical isolator, the method comprising:
- generating vaporized hydrogen peroxide through photocatalysis by activating a photocatalytic material, which covers a plate (18), through UV radiation in order to transform the humidity of the air present in the isolated chamber (2) into vaporized hydrogen peroxide; and
- introducing the vaporized hydrogen peroxide into the isolated chamber (2) through a forced ventilation system (14) comprising a pneumatic circuit (20) and a fan (21) positioned in the pneumatic circuit (20) to circulate the air between the isolated chamber (2) and the vaporized hydrogen peroxide generator (13); the method being **characterized in that** said plate is a metal plate (18) and **in that** it comprises:
- measuring the vaporized hydrogen peroxide concentration in the isolated chamber (2) by means of a spectroscopy gas analyser (15) communicating with the isolated chamber (2);
- adjusting the generation of vaporized hydrogen peroxide as a function of measured values (CMeas) of the vaporized hydrogen peroxide concentration and of at least one reference value (CRef) of the vaporized hydrogen peroxide concentration; and
- controlling the power of said UV radiation source (19) so as to adjust the intensity of the UV radiation and, hence, the quantity of vaporized hydrogen peroxide generated and introduced into the isolated chamber (2).

9. The method according to claim 8 and comprising, before generating vaporized hydrogen peroxide and introducing the latter into the isolated chamber (2):
- allowing air to circulate in the isolated chamber (2) for a predetermined amount of time through a further forced ventilation system (3);
- during the circulation of air, reducing the presence of particulate matter in the isolated chamber (2) by means of HEPA air filtering means (4) arranged in the further forced ventilation system (3); and
- at the end of said predetermined amount of time, closing the further forced ventilation system (3) so as to prevent air from circulating in the isolated chamber (2).

## Patentansprüche

1. Vorrichtung zum Einbringen von verdampftem Wasserstoffperoxid mit einer Konzentration kleiner oder gleich 10 PPM in eine isolierte Kammer und insbesondere in eine Kammer eines Isolators, beispielsweise eines pharmazeutischen Isolators, wobei die Vorrichtung umfasst: einen Generator (13) für verdampftes Wasserstoffperoxid, der durch Photokatalyse arbeitet und mindestens eine Platte (18) mit einer Beschichtung aus photokatalytischem Material, das durch UV-Strahlung aktiviert werden kann, um die Feuchtigkeit der in der isolierten Kammer (2) vorhandenen Luft in verdampftes Wasserstoffperoxid umzuwandeln, und mindestens eine UV-Strahlungsquelle (19) umfasst, die so angeordnet ist, dass sie die Beschichtung mit UV-Strahlung bestrahlt und so die Photokatalyse aktiviert; und ein Zwangsbelüftungssystem (14), das geeignet ist, das verdampfte Wasserstoffperoxid in die isolierte Kammer (2) einzuführen, und das einen pneumatischen Kreislauf (20), der geeignet ist, eine geschlossene Verbindung zwischen der isolierten Kammer (2) und dem Generator (13) für verdampftes Wasserstoffperoxid herzustellen, und ein Gebläse (21) umfasst, das in dem pneumatischen Kreislauf (20) angeordnet ist, um die Luft zwischen der isolierten Kammer (2) und dem Generator (13) für verdampftes Wasserstoffperoxid umzuwälzen; wobei die Vorrichtung **dadurch gekennzeichnet ist, dass** die Platte eine Metallplatte (1) ist und dass sie einen Spektroskopie-Gasanalysator (15) umfasst, der geeignet ist, mit der isolierten Kammer (2) zu kommunizieren, um die Konzentration des verdampften Wasserstoffperoxids in der isolierten Kammer (2) zu messen, sowie Steuermittel (16), die so konfiguriert sind, dass sie den Generator (13) für verdampftes Wasserstoffperoxid als Funktion von gemessenen Werten (CMeas) der Konzentration des verdampften Wasserstoffperoxids und von mindestens einem Referenzwert (CRef) der Konzentration des verdampften Wasserstoffperoxids steuern und die Leistung der UV-Strahlungsquelle (19) steuern, um die Intensität der UV-Strahlung und damit die Menge des erzeugten und in die isolierte Kammer (2) eingeleiteten verdampften Wasserstoffperoxids einzustellen.

2. Vorrichtung nach Anspruch 1, wobei das photokatalytische Material aus Titandioxid besteht.

3. Vorrichtung nach Anspruch 1 oder 2, wobei der Spektroskopie-Gasanalysator (15) eine CRDS-Spektroskopie durchführt.

4. Isolator, insbesondere ein pharmazeutischen Isolator, mit einer isolierten Kammer (2) und einer Vorrichtung (12) zum Einleiten von verdampftem Wasserstoffperoxid mit einer Konzentration von höchstens 10 PPM in die isolierte Kammer (2) nach einem der Ansprüche 1 bis 3.

5. Isolator nach Anspruch 4 und mit einem weiteren Zwangsbelüftungssystem (3), das so ausgelegt ist, dass Luft in der isolierten Kammer (2) zirkulieren kann, und das HEPA-Luftfiltermittel (4), um das Vorhandensein von Partikeln in der isolierten Kammer (2) zu begrenzen, Ventile (10, 11), um die Zirkulation der Luft in der isolierten Kammer (2) zu ermöglichen oder zu verhindern, und eine elektronische Steuereinheit (25) umfasst, die so konfiguriert ist, dass sie die Ventile (10, 11) schließt, bevor sie die Vorrichtung (12) aktiviert.

6. Isolator nach Anspruch 5, wobei das weitere Zwangsbelüftungssystem (3) einen Zuführungskanal (6) und einen Rückführungskanal (7) umfasst, um Luft in die isolierte Kammer (2) einzuführen bzw. aus dieser abzuführen, wobei die HEPA-Luftfiltereinrichtung (4) mindestens zwei HEPA-Filter (8, 9) umfassen, die vorzugsweise zur Klasse H14 gehören und von denen ein erster in der Zufuhrleitung (6) und der zweite in der Rückführleitung (7) angeordnet ist, und die Ventile zwei Ventile (10, 11) umfassen, von denen ein erstes in der Zufuhrleitung (6) und das zweite in der Rückführleitung (7) angeordnet ist.

7. Isolator nach einem der Ansprüche 4 bis 6 mit einer weiteren Vorrichtung (26) zum Einleiten von verdampftem Wasserstoffperoxid in die isolierte Kammer (2) mit einer Konzentration von 400 PPM oder mehr, um eine mikrobiologische Dekontamination der isolierten Kammer (2) zu ermöglichen.

8. Verfahren zum Einbringen von verdampftem Wasserstoffperoxid mit einer Konzentration von weniger als oder gleich 10 PPM in eine isolierte Kammer, insbesondere in eine Kammer eines Isolators, zum Beispiel eines pharmazeutischen Isolators, wobei das Verfahren umfasst:
- Erzeugung von verdampftem Wasserstoffperoxid durch Photokatalyse, indem ein photokatalytisches Material, das eine Platte (18) bedeckt, durch UV-Strahlung aktiviert wird, um die Feuchtigkeit der in der isolierten Kammer (2) vorhandenen Luft in verdampftes Wasserstoffperoxid umzuwandeln; und
- Einführen des verdampften Wasserstoffperoxids in die isolierte Kammer (2) durch ein System der Zwangsbelüftung (14), das einen pneumatischen Kreislauf (20) und ein Gebläse (21) umfasst, das in dem pneumatischen Kreislauf (20) angeordnet ist, um die Luft zwischen der isolierten Kammer (2) und dem Generator für verdampftes Wasserstoffperoxid (13) zirkulieren zu lassen; wobei das Verfahren **dadurch gekennzeichnet ist, dass** die Platte eine Metallplatte (18) ist, und dass es umfasst:
- Messen der Konzentration des verdampften Wasserstoffperoxids in der isolierten Kammer (2) mit Hilfe eines Spektroskopie-Gasanalysators (15), der mit der isolierten Kammer (2) in Verbindung steht;
- Einstellen der Erzeugung von verdampftem Wasserstoffperoxid in Abhängigkeit von gemessenen Werten (CMeas) der Konzentration an verdampftem Wasserstoffperoxid und von mindestens einem Referenzwert (CRef) der Konzentration an verdampftem Wasserstoffperoxid, und
- Steuerung der Leistung der UV-Strahlungsquelle (19), um die Intensität der UV-Strahlung und damit die Menge des erzeugten und in die isolierte Kammer (2) eingeleiteten verdampften Wasserstoffperoxids einzustellen.

9. Verfahren nach Anspruch 8, das vor der Erzeugung von verdampftem Wasserstoffperoxid und dem Einleiten desselben in die isolierte Kammer (2) Folgendes vorsieht:
- Zirkulieren lassen von Luft in der isolierten Kammer (2) für eine bestimmte Zeit durch ein weiteres Zwangsbelüftungssystem (3);
- Reduzieren des Vorhandenseins von Partikeln in der isolierten Kammer (2) während der Luftzirkulation mit Hilfe von HEPA-Luftfiltermitteln (4), die in dem weiteren Zwangsbelüftungssystem (3) angeordnet sind, und
- am Ende der vorbestimmten Zeitdauer, Schließen des weiteren Zwangsbelüftungssystems (3), um zu verhindern, dass Luft in der isolierten Kammer (2) zirkuliert.

## Revendications

1. Appareil pour introduire du peroxyde d'hydrogène vaporisé à une concentration inférieure ou égale à 10 ppm dans une chambre isolée, et en particulier dans une chambre d'un isolateur, par exemple un isolateur pharmaceutique, l'appareil comprenant : un générateur de peroxyde d'hydrogène vaporisé (13), qui fonctionne par photocatalyse et comprend au moins une plaque (18) ayant un revêtement en un matériau photocatalytique activable par un rayonnement UV, afin de transformer l'humidité de l'air présent dans la chambre isolée (2) en peroxyde d'hydrogène vaporisé, et au moins une source de rayonnement UV (19) disposée de façon à irradier un rayonnement UV sur ledit revêtement, en activant ainsi la photocatalyse ; et un système de ventilation forcée (14), qui convient pour introduire le peroxyde d'hydrogène vaporisé dans la chambre isolée (2) et comprend un circuit pneumatique (20) convenant pour établir une communication en circuit fermé entre la chambre isolée (2) et le générateur de peroxyde d'hydrogène vaporisé (13) et un ventilateur (21) positionné dans le circuit pneumatique (20) pour faire circuler l'air entre la chambre isolée (2) et le générateur de peroxyde d'hydrogène vaporisé (13) ; l'appareil étant **caractérisé en ce que** ladite plaque est une plaque métallique (1) et **en ce qu'**il comprend un analyseur de gaz par spectroscopie (15), qui convient pour communiquer avec la chambre isolée (2) de façon à mesurer la concentration du peroxyde d'hydrogène vaporisé dans la chambre isolée (2), et des moyens de commande (16), qui sont configurés pour commander le générateur de peroxyde d'hydrogène vaporisé (13) en fonction de valeurs mesurées (CMeas) de la concentration de peroxyde d'hydrogène vaporisé et d'au moins une valeur de référence (CRef) de la concentration de peroxyde d'hydrogène vaporisé et pour commander la puissance de ladite source de rayonnement UV (19) de façon à ajuster l'intensité du rayonnement UV et, par conséquent, la quantité de peroxyde d'hydrogène vaporisé généré et introduit dans la chambre isolée (2).

2. Appareil selon la revendication 1, dans lequel ledit matériau photocatalytique consiste en dioxyde de titane.

3. Appareil selon la revendication 1 ou 2, dans lequel ledit analyseur de gaz par spectroscopie (15) effectue une spectroscopie CRDS.

4. Isolateur, en particulier isolateur pharmaceutique, comprenant une chambre isolée (2) et un appareil (12) pour introduire du peroxyde d'hydrogène vaporisé à une concentration inférieure ou égale à 10 ppm dans le chambre isolée (2) selon l'une quelconque des revendications 1 à 3.

5. Isolateur selon la revendication 4 et comprenant un autre système de ventilation forcée (3), qui est conçu pour permettre à de l'air de circuler dans la chambre isolée (2) et comprend des moyens de filtration de l'air HEPA (4) pour limiter la présence de matières particulaires dans la chambre isolée (2), des vannes (10, 11) pour permettre ou empêcher ledit air de circuler dans la chambre isolée (2), et une unité de commande électronique (25) configurée pour fermer les vannes (10, 11) avant l'activation dudit appareil (12).

6. Isolateur selon la revendication 5, dans lequel ledit autre système de ventilation forcée (3) comprend une conduite de délivrance (6) et une conduite de retour (7) pour introduire de l'air dans et extraire de l'air depuis ladite chambre isolée (2), respectivement, lesdits moyens de filtration de l'air HEPA comprenant au moins deux filtres HEPA (8, 9), de préférence appartenant à la classe H14, qui sont disposés le premier dans la conduite de délivrance (6) et le deuxième dans la conduite de retour (7), et lesdites vannes comprennent deux vannes (10, 11) qui sont disposées la première dans la conduite de délivrance (6) et la deuxième dans la conduite de retour (7).

7. Isolateur selon l'une quelconque des revendications 4 à 6 et comprenant un autre appareil (26) pour introduire dans la chambre isolée (2) du peroxyde d'hydrogène vaporisé à une concentration supérieure ou égale à 400 ppm, pour permettre une décontamination microbiologiue de la chambre isolée (2).

8. Procédé pour introduire du peroxyde d'hydrogène vaporisé à une concentration inférieure ou égale à 10 ppm dans une chambre isolée, et en particulier dans une chambre d'un isolateur, par exemple un isolateur pharmaceutique, le procédé comprenant :
- la génération de peroxyde d'hydrogène vaporisé par l'intermédiaire d'une photocatalyse par activation d'un matériau photocatalytique, qui couvre une plaque (18), par l'intermédiaire d'un rayonnement UV afin de transformer l'humidité de l'air présent dans la chambre isolée (2) en peroxyde d'hydrogène vaporisé ; et
- l'introduction du peroxyde d'hydrogène vaporisé dans la chambre isolée (2) par l'intermédiaire d'un système de ventilation forcée (14) comprenant un circuit pneumatique (20) et un ventilateur (21) positionné dans le circuit pneumatique (20) pour faire circuler l'air entre la chambre isolée (2) et le générateur de peroxyde d'hydrogène vaporisé (13) ;
le procédé étant **caractérisé en ce que** ladite plaque est une plaque métallique (18) et **en ce qu'**il comprend :
- la mesure de la concentration de peroxyde d'hydrogène vaporisé dans la chambre isolée (2) au moyen d'un analyseur de gaz par spectroscopie (15) communiquant avec la chambre isolée (2) ;
- l'ajustement de la génération de peroxyde d'hydrogène vaporisé en fonction de valeurs mesurées (CMeas) de la concentration de peroxyde d'hydrogène vaporisé et d'au moins une valeur de référence (CRef) de la concentration de peroxyde d'hydrogène vaporisé ; et
- la commande de la puissance de ladite source de rayonnement UV (19) de façon à ajuster l'intensité du rayonnement UV et, par conséquent, la quantité de peroxyde d'hydrogène vaporisé généré et introduit dans la chambre isolée (2).

9. Procédé selon la revendication 8 et comprenant, avant la génération de peroxyde d'hydrogène vaporisé et l'introduction de ce dernier dans la chambre isolée (2) ;
- le fait de laisser l'air circuler dans la chambre isolée (2) pendant une quantité de temps prédéterminée par l'intermédiaire d'un autre système de ventilation forcée (3) ;
- durant la circulation de l'air, la réduction de la présence de matières particulaires dans la chambre isolée (2) au moyen de moyens de filtration de l'air HEPA (4) disposés dans l'autre système de ventilation forcée (3) ; et
- à la fin de ladite quantité de temps prédéterminée, la fermeture de l'autre système de ventilation forcée (3) de façon à empêcher l'air de circuler dans la chambre isolée (2).
